# EUROPÄISCHE PATENTSCHRIFT

(11) **EP 0 633 750 B1**
(45) Veröffentlichungstag und Bekanntmachung des Hinweises auf die Patenterteilung: **27.05.1998**
(21) Anmeldenummer: 93902013.7
(22) Anmeldetag: 25.01.1993
(51) Int. Cl.: A61B 17/58

(54) **GEGENSPANNSCHEIBE FÜR DIE PLATTENOSTEOSYNTHESE**
STRAIN WASHER FOR PLATE OSTEOSYNTHESIS
DISQUE DE CONTRAINTE POUR L'OSTEOSYNTHESE PAR PLAQUE

(43) Veröffentlichungstag der Anmeldung: 18.01.1995
(73) Patentinhaber: Synthes AG, Chur, 7002 Chur (CH)
(72) Erfinder: KLAUE, Kaj, CH-3005 Bern (CH); MAST, Jeffrey, W., Grosse Pointe Park, MI 48230 (US)
(74) Vertreter: Lusuardi, Werther Giovanni, Dr.
(86) Internationale Anmeldenummer: CH9300018
(87) Internationale Veröffentlichungsnummer: WO9416634

(56) Entgegenhaltungen:
- EP-A- 0 301 489
- EP-A- 0 360 139
- EP-A- 0 410 309
- EP-A- 0 507 162
- WO-A-90/12547
- DE-A- 3 027 148
- DE-A- 3 509 417
- US-A- 5 057 111
- US-A- 5 129 899

## Beschreibung

Die Erfindung betrifft eine Gegenspannscheibe zur rigiden Fixation von Knochenschrauben an eine Knochenplatte gemäss dem Oberbegriff des Anspruchs 1 sowie eine Fixationsvorrichtung mit einer solchen Gegenspannscheibe.
Die meisten in der Osteosynthese verwendeten Knochenplatten werden üblicherweise nur mit Hilfe von Knochenschrauben direkt am Knochen befestigt und einzig durch die zwischen Knochenplatte und Knochen resultierende Reibungskraft am Knochen gehalten. Da die Knochenschrauben lediglich im Knochen verankert sind, besteht keine rigide Verankerung derselben mit der Knochenplatte.
Eine Lockerung der Schrauben im Knochen oder auch eine Resorption des Knochens kann somit leicht zu einer Lockerung der Knochenplatte selbst führen.
Es ist zwar bei der Plattenosteosynthese schon bekannt Knochenschrauben, welche den Knochen vollständig durchdringen, an ihrem, dem Schraubenkopf abgewandten Ende mittels einer Mutter zu sichern. Auch diese Methode bewirkt jedoch keine direkte Fixation zwischen Schrauben und Platte sondern lediglich eine Kompression, des zwischen der Mutter und der Platte liegenden, von der Schraube durchbohrten Knochens.
Bei einer grösseren Anzahl von Indikationen ist es jedoch wünschenswert eine rigide Fixation zwischen Knochenschraube und Knochenplatte herzustellen, um eine spätere Lockerung zu vermeiden. Zu diesem Zweck ist es bereits aus der EP-A 340 223 bekannt, den Kopf der Knochenschraube in einer speziell ausgebildeten, konische Bohrlöcher aufweisenden Knochenplatte mittels Reibungshaftung allein zu blockieren. Bei dieser bekannten Vorrichtung sind aber spezielle Knochenplatten mit Bohrungen definierter Konizität und spezielle Knochenschrauben mit entsprechend ausgebildeten Schraubenköpfen notwendig. Eine Verschwenkbarkeit der Knochenschraube ist nur mit Hilfe zusätzlicher aufwendiger Massnahmen, z.B. mittels eines separaten sphärischen Einsatzes möglich. Es ist ebenfalls bereits aus der EP-A 340 223 bekannt, die Unterseite von Knochenplatten mit Erhebungen zu versehen um die am Knochen zur Anlage kommende Kontaktfläche zwecks Verbesserung der Vaskularität zu reduzieren. Erhebungen und Knochenplatte bilden jedoch eine Einheit, die nur zusammen verwendet werden kann und keine individuellen Ausgestaltungen erlaubt. Im weiteren sind die am Knochen zur Anlage kommenden Erhebungen am Rand der Platte angeordnet, d.h. in einem grösseren Abstand vom Ort des Durchtritts der Knochenschraube. Dadurch ergibt sich eine zusätzliche Schädigung, bzw. Beeinträchtigung der Knochenoberfläche.
Schliesslich ist aus der EP-A1 0 301 489 eine Knochenschraube mit einer Gegenspannscheibe bekannt, welche sich sowohl in Richtung des Knochens als auch in Richtung der Knochenplatte verjüngt.
Nachteilig bei dieser bekannten - symmetrischen - Gegenspannscheibe ist der Umstand, dass der Umfang ihres unteren und oberen Abschnitts genau gleich gross ist.

Hier will die Erfindung Abhilfe schaffen.

Der Erfindung liegt die Aufgabe zugrunde eine Gegenspannscheibe für die Plattenosteosynthese zu schaffen, welche mit jedem beliebigen Typ von Knochenplatten und Knochenschrauben, sowie wahlweise an jeder gewünschten Position (Bohrung) der Knochenplatte eingesetzt werden kann und dabei gleichzeitig als Abstandshalter zur Verringerung der Kontaktfläche zwischen Knochenplatte und Knochen wirkt.

Die Erfindung löst die Aufgabe mit einer Gegenspannscheibe gemäss dem kennzeichnenden Teil des Anspruchs 1, sowie einer Fixationsvorrichtung gemäss Anspruch 9. Bevorzugte Ausbildungen der Erfindungen sind in den abhängigen Ansprüchen definiert.

Der obere Abschnitt der erfindungsgemässen Gegenspannscheibe kann auf verschiedene Arten geformt sein, sofern dieser nur in die Bohrung der Knochenplatte einführbar ist; vorzugsweise wird jedoch ein elliptisches oder rechteckähnliches Querschnittsprofil gewählt um eine drehfeste Blockierung in der Bohrung der Knochenplatte zu erzielen. Im weiteren wird eine formschlüssige Verbindung zwischen oberem Abschnitt der Gegenspannscheibe und unterem Abschnitt der Bohrung der Knochenplatte bevorzugt. Die axiale Höhe des oberen Abschnitts der Gegenspannscheibe ist zweckmässigerweise so zu dimensionieren, dass er vollständig in der Bohrung der Knochenplatte Platz findet.

Das Innengewinde der erfindungsgemässen Gegenspannscheibe muss selbstverständlich dem standardisierten Aussengewinde der zu verwendenden Knochenschrauben entsprechen.

Bei einem Einsatz der erfindungsgemässen Gegenspannscheibe mit Knochenplatten, welche auch kreisrunde Bohrungen aufweisen, kann der untere Abschnitt nicht wie bei elliptischen Bohrungen drehfest darin eingeführt werden. Um diesem Mangel abzuhelfen, ist der obere Abschnitt der Gegenspannscheibe mindestens an der mit der Knochenplatte zur Anlage kommenden Oberfläche mit einer Aufrauhung oder Strukturierung versehen. Eine weitere Verbesserung der Drehfestigkeit kann dadurch erreicht werden, dass auch die Bohrungen der Knochenplatte mindestens an den mit der Gegenspannscheibe zur Anlage kommenden Oberfläche mit einer Aufrauhung oder Strukturierung versehen werden. Dank dieser oder analoger Massnahmen (z.B. korrespondierende Erhebungen und Vertiefungen in den beiden zur Anlage kommenden Oberflächen) kann die sonst fehlende Rotationssicherung trotzdem erreicht werden.

Die Vorteile der erfindungsgemässen Gegenspannscheibe sind vorallem in ihrer universellen Anwendbarkeit zu erblicken, da sie praktisch mit jedem beliebigen Typ von Knochenplatte und Knochenschraube kombiniert werden kann. Sie gestattet zudem eine gezielte, auf einzelne Positionen (Bohrungen) der Knochenplatte, bzw. der darin eingeführten Knochenschrauben beschränkte Applikation. Die Verwendung der erfindungsgemässen Gegenspannscheibe kann somit noch intraoperativ in jeder beliebigen Operationsphase erfolgen.

Ein weiterer Vorteil der erfindungsgemässen Gegenspannscheibe ist in ihrer Wirkung als Abstandshalter zwischen der Knochenplatte und dem Knochen zu sehen. Die reduzierte Knochenkontaktfläche gestattet eine verbesserte Durchblutung und damit eine raschere Heilung. Dabei ist der Umstand von besonderer Bedeutung, dass die Knochenanlagefläche der erfindungsgemässen Gegenspannscheibe direkt am Ort des Durchtritts der Knochenschraube angeordnet ist; es ergibt sich somit einerseits eine kaum vergrösserte Beeinträchtigung der Knochenoberfläche in diesem ohnehin bereits belasteten Bereich und anderseits eine Verstärkung der mechanischen Festigkeit der als Abstandshalter wirkenden Gegenspannscheibe im knochennahen Auflagebereich.

Im weiteren ist es dank der, mittels der erfindungsgemässen Gegenspannscheibe erzielten, rigiden Fixation der Knochenschraube an der Knochenplatte möglich, letztere in einem definierten Abstand vom Knochen anzuordnen, so dass ein sogenannter "ultra-low profile external fixator" entsteht.

Die rigide Fixation verhindert ferner das unerwünschte Auftreten von Deformationen wie sie bei axialer Kompression durch Rotation der Schrauben auftreten können.

Die Gegenspannscheibe gestattet auch eine gewisse Energiespeicherung, wenn eine Kompression auf die Fraktur augeübt wird. Somit ist es möglich, eine längerdauernde Kompressionswirkung auszuüben. Sie entlastet auch die Schrauben, die nach konventioneller Art durch ein Plattenloch, z.B. als Zugschraube oder als sogenannte "Plattenzugschraube" positioniert sind.

Im weiteren gestattet die Gegenspannscheibe auch eine gewisse seitliche Verschwenkbarkeit der Knochenschraube gegenüber der Mittelachse des Plattenlochs, ohne dass dazu zusätzliche Hilfsmittel vorgesehen werden müssen.

Die Gegenspannscheibe kann bei folgenden Indikationen Verwendung finden:
a) Bei osteoporotischen Knochen oder Knochen mit dünner Kortikalis (da eine rigide Fixation zwischen Platte und Schrauben besteht, kann eine frühzeitige Lockerung, wie sie sich durch zyklische Belastungen ergeben kann, vermieden werden);
b) Bei einem Knochendefekt in der nahen Kortikalis, wie er beispielsweise bei einem Schraubenbruch im Knochen auftreten kann.

Die Erfindung wird nun anhand der Zeichnungen, welche bevorzugte Ausführungsformen darstellen, im Einzelnen erläutert.

Es zeigt:
Fig. 1 eine explosionsartige, perspektivische Ansicht einer Knochenplatte mit Knochenschraube und erfindungsgemässer Gegenspannscheibe;
Fig. 2 einen Querschnitt senkrecht zur Plattenlängsachse durch die Knochenplatte mit eingesetzter Knochenschraube und erfindungsgemässer Gegenspannscheibe nach Fig. 1.
Fig. 3 einen Längsschnitt durch eine Knochenplatte mit eingesetzter Knochenschraube und einer Variante der erfindungsgemässen Gegenspannscheibe;
Fig. 4 einen Querschnitt senkrecht zur Plattenlängsachse durch die Knochenplatte mit eingesetzter Knochenschraube und erfindungsgemässer Gegenspannscheibe nach Fig. 3;
Fig. 5 eine Aufsicht auf die Knochenplatte mit eingesetzter Knochenschraube und Gegenspannscheibe nach Fig. 3;
Fig. 6 einen Längsschnitt durch eine modifizierte, erfindungsgemässe Gegenspannscheibe für eine abgewinkelte Knochenschraube; und
Fig. 7 einen Längsschnitt durch eine weitere Modifikation einer erfindungsgemässen Gegenspannscheibe.

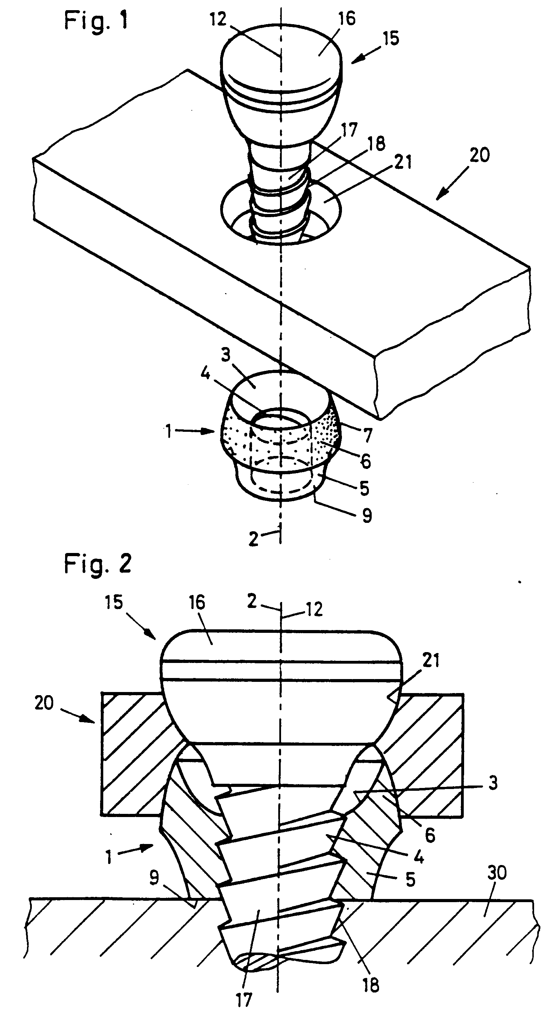

Wie in Fig. 1 und 2 dargestellt besteht die erfindungsgemässe Gegenspannscheibe 1 im wesentlichen aus einem einstückigen Grundkörper 5,6 mit einer zentralen Achse 2, einer zentralen Bohrung 3 mit einein zum Aussengewinde 18 der Knochenschraube 15 passenden Innengewinde 4, einem unteren Abschnitt 5, einem oberen Abschnitt 6 und einer Knochenanlagefläche 9 zum Knochen 30.
Der obere Abschnitt 6 ist derart geformt, dass er von unten in die Bohrung 21 der Knochenplatte 20 im wesentlichen formschlüssig einführbar ist. Da das Bohrloch 21 sich von unten gegen oben konisch verjüngt, ist der obere Abschnitt 6 ebenfalls als sich verjüngender Konus ausgebildet. Die Oberfläche 7 des Abschnitts 6 ist aufgerauht, damit beim knochenfernen Anziehen der Gegenspannscheibe 1 eine gewisse Rotationssicherheit innerhalb des Bohrlochs 21 erzielt wird.
Der mittlere Umfang des unteren Abschnitts 5 ist kleiner als der mittlere Umfang des oberen Abschnitts 6. Diese Ausgestaltung ist von grosser Wichtigkeit, weil dadurch eine relativ geringe Auflagefläche 9 auf dem Knochen entsteht.
Beim Eindrehen des Schaftes 17 der Knochenschraube 15 in den Knochen 30 kommt der Schraubenkopf 16 im Bohrloch 21 - wie in Fig. 2 dargestellt - zur Anlage ohne dass der Schraubenkopf 16 die Gegenspannscheibe 1 berührt, was für deren Funktion wesentlich ist.

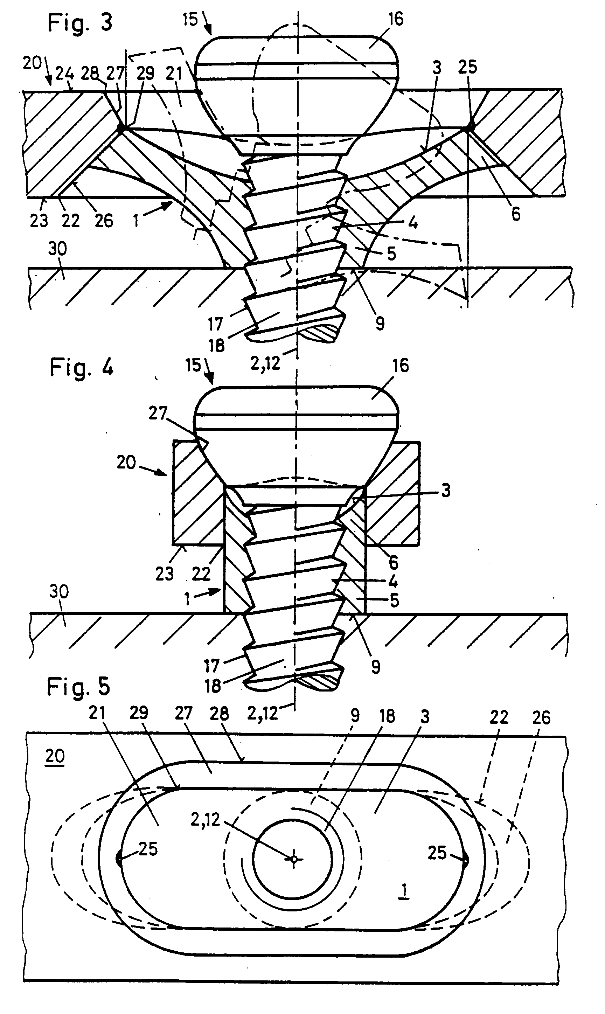

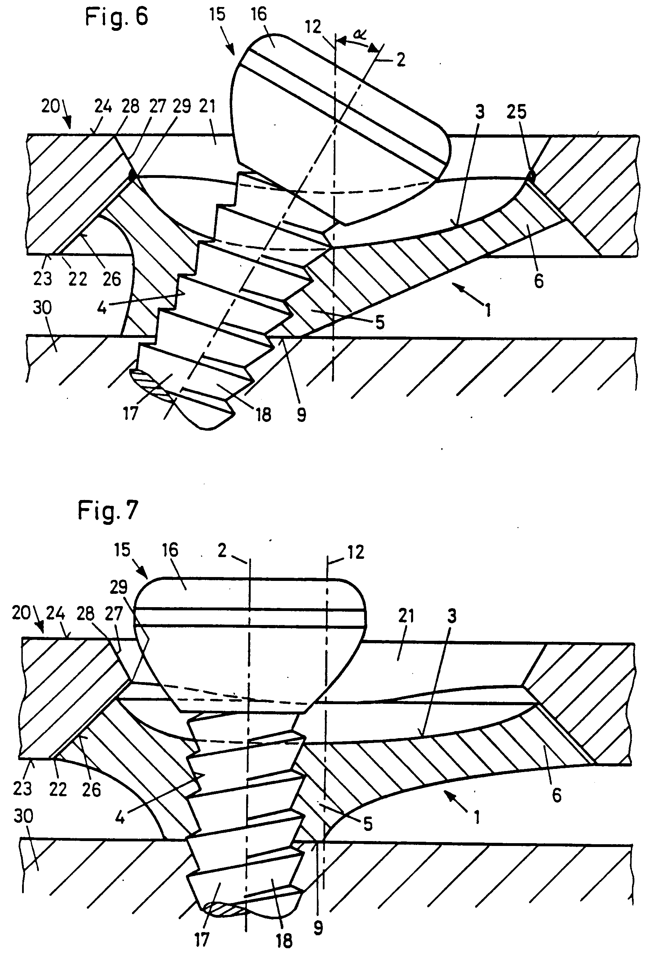

In den Fig. 3 - 5 ist eine Variante der Gegenspannscheibe 1 dargestellt, welche den Bedürfnissen für eine Knochenplatte 20 mit einem in deren Längsrichtung verlängerten Bohrloch 21 angepasst worden ist.
Die Gegenspannscheibe 1 ist bei dieser Ausführungsform nicht mehr rotationssymmetrisch, wie bei der Ausführung nach den Fig. 1 und 2 sondern elliptoid ausgebildet, um sich der im wesentlichen ovalen Geometrie des Bohrloches 21 anpassen zu können. Wie aus Fig. 5 hervorgeht, entspricht die Oberflächengeometrie des oberen Abschnitts 6 der Gegenspannscheibe 1 nicht genau dem Rand 22 des Bohrloches 21 an der Unterseite 23 der Knochenplatte 20 sondern ist diesbezüglich unterdimensioniert. Durch diese Massnahme ist es möglich die Gegenspannscheibe 1 nicht nur von der Unterseite 23 der Knochenplatte 20 in das Bohrloch 21 einzuführen, sondern auch von der Oberseite 24 des Bohrloch 21, was am zweckmässigsten so geschieht, dass die Gegenspannscheibe 1 provisorisch auf die Knochenschraube 15 aufgeschraubt wird und mit Hilfe derselben durch das Bohrloch 21 geführt wird. Zu diesem Zweck ist die Achse 2 der Gegenspannscheibe 1 in Längsrichtung der Knochenplatte 20 etwas zu neigen (siehe die gestrichelten Umrisse in Fig. 3), bis sie über die elastischen Nippel (oder Wülste) 25 nach unten gedrückt und dann nach Geradestellung der Achse 2 wieder ins Bohrloch 21 zurückgezogen und gegen den sich verjüngenden konischen Abschnitt 26 des Bohrloches 21 zur Anlage gebracht werden kann. Die Nippel 25 sind an zwei entgegengesetzten Seiten in Längsrichtung des Bohrloches 21 angebracht und zwar an der Stelle des Bohrloches 21 mit dem geringsten Umfang 29. Das Bohrloch 21 verbreitert sich von diesem geringsten Umfang 29 aus nach oben wiederum in einem konischen Abschnitt 27 bis zum Rand 28 des Bohrloches 21 an der Oberseite 24 der Knochenplatte 20.
Die Funktion der Nippel 25 besteht darin, die Gegenspannscheibe 1 nach dem oben beschriebenen knochenfernem Zurückziehen ins Bohrloch 21 (oder nach deren Eindrücken von unten) provisorisch festzuklemmen, so dass die Gegenspannscheibe 1 auch dann nicht aus den Bohrloch 21 fällt, wenn die Knochenschraube 15 intraoperativ entfernt wird.

In Fig. 6 ist eine weitere Variante der Gegenspannscheibe 1 dargestellt, welche eine Neigung der Achse 2 der Knochenschraube 15 gegenüber der Achse 12 des hier ebenfalls als Längsloch ausgebildeten Bohrlochs 21 zulässt. Zu diesem Zweck ist die Cegenspannscheibe 1 nicht nur oval, sondern zusätzlich auch noch asymmetrisch ausgebildet, damit bei einer Anlage gegen den sich verjüngenden konischen Abschnitt 26 des Bohrloches 21 eine Neigung der Knochenschraube 15 um den Winkel α resultiert, der bis zu 30° betragen kann. Auch bei dieser Ausführungsform sind Nippel (oder Wülste) 25 vorgesehen, welche ein Herausfallen aus dem Bohrloch 21 der noch nicht fixierten Gegenspannscheibe 1 verhindern.

Schliesslich ist in Fig. 7 eine weitere Ausführungsform der Gegenspannscheibe 1 dargestellt, welche für eine Knochenplatte 20 modifiziert wurde, bei welcher mindestens eines der Bohrlöcher 21 als sogenanntes Gleitloch ausgebildet ist und demzufolge die Knochenschraube 15 exzentrisch im Bohrloch 21 zu liegen kommt. Demzufolge ist auch die Bohrung 3 in der Gegenspannscheibe 1 exzentrisch angeordnet. Das Prinzip eines solchen Gleitlochs ist in der CH-A5 650 915 KLAUE im Detail beschrieben. Im übrigen ist die Gegenspannscheibe 1 analog zu den oben beschriebenen Ausführungsformen ausgestaltet.

## Patentansprüche

1. Gegenspannscheibe (1) zur rigiden Fixation von Knochenschrauben (15) an eine, mehrere Bohrungen (21) aufweisende Knochenplatte (20), wobei die zylindrische Gegenspannscheibe (1) eine zentrale Achse (2), eine zentrale Bohrung (3) mit einem eine Knochenschraube (15) aufnehmbaren Innengewinde (4), einen unteren Abschnitt (5) mit einer Knochenanlagefläche (9) und einen oberen Abschnitt (6) aufweist, wobei der obere Abschnitt (6), bei Verwendung der Gegenspannscheibe, in der Bohrung (21) der Knochenplatte (20) zum Anschlag gebracht wird, dadurch gekennzeichnet, dass der mittlere Umfang des unteren Abschnitts (5) kleiner ist als der mittlere Umfang des oberen Abschnitts (6).

2. Gegenspannscheibe (1) nach Anspruch 1, dadurch gekennzeichnet, dass das Innengewinde (4) vollständig im unteren Abschnitt (5) untergebracht ist.

3. Gegenspannscheibe (1) nach Anspruch 1 oder 2, dadurch gekennzeichnet, dass sie sich vom oberen Abschnitt (6) zum unteren Abschnitt (5) hin, entweder kontinuierlich oder nicht kontinuierlich, verjüngt.

4. Gegenspannscheibe (1) nach einem der Ansprüche 1 bis 3, dadurch gekennzeichnet, dass der obere Abschnitt (6) mindestens an der mit der Knochenplatte (20) zur Anlage kommenden Oberfläche (7) eine Aufrauhung oder Strukturierung aufweist um die Drehfestigkeit zu erhöhen.

5. Gegenspannscheibe (1) nach einem der Ansprüche 1 bis 4, dadurch gekennzeichnet, dass sie symmetrisch ausgebildet ist.

6. Gegenspannscheibe (1) nach einem der Ansprüche 1 bis 4, dadurch gekennzeichnet, dass sie asymmetrisch ausgebildet ist.

7. Gegenspannscheibe (1) nach einem der Ansprüche 1 bis 6, dadurch gekennzeichnet, dass die Knochenanlagefläche (9) kleiner ist als die durchschnittliche zur Achse (2) senkrecht stehende Querschnittsfläche im oberen Abschnitt (6).

8. Gegenspannscheibe (1) nach einem der Ansprüche 1 - 7, dadurch gekennzeichnet, dass sie elliptoid ausgebildet ist.

9. Fixationsvorrichtung mit mindestens einer Gegenspannscheibe (1) nach einem der Ansprüche 1 bis 8, einer Knochenplatte (20) mit einer Unterseite (23) und einer Oberseite (24), welche von Bohrungen (21) durchsetzt sind, sowie mindestens eine Knochenschraube (15) mit einem Kopf (16), einem Schaft (17) und einem Aussengewinde (18), wobei bei Fixierung der Fixationsvorrichtung der obere Abschnitt (6) der Gegenspannscheibe (1) in der Bohrung (21) zum Anschlag gebracht wird.

10. Fixationsvorrichtung nach Anspruch 9, dadurch gekennzeichnet, dass mindestens eine der Bohrungen (21) einen sich nach der Unterseite (23) erweiterenden konischen Abschnitt (26) aufweist, welcher, formschlüssig mit der Geometrie des oberen Abschnitts (6) der Gegenspannscheibe (1) geformt ist und bei Fixierung der Fixationsvorrichtung zur Anlage des oberen Abschnitts (6) der Gegenspannscheibe (1) gebracht wird.

11. Fixationsvorrichtung nach Anspruch 9 oder 10, dadurch gekennzeichnet, dass mindestens eine der Bohrungen (21) der Knochenplatte (20) mindestens an den mit der Gegenspannscheibe (1) zur Anlage kommenden Oberfläche eine Aufrauhung oder Strukturierung aufweist um die Drehfestigkeit zu erhöhen.

12. Fixationsvorrichtung nach Anspruch 11, dadurch gekennzeichnet, dass die Aufrauhung oder Strukturierung in Form eines sich verjüngenden konischen Abschnittes (26) der Bohrung (21) ausgebildet ist.

13. Fixationsvorrichtung nach einem der Ansprüche 9 bis 12, dadurch gekennzeichnet, dass mindestens eine der Bohrungen (21) der Knochenplatte (20) an ihrem engsten Umfang (29) mit als elastische Nippel (25) ausgebildeten Sicherungselementen für die Gegenspannscheibe (1) versehen ist.

14. Fixationsvorrichtung nach einem der Ansprüche 9 bis 13, dadurch gekennzeichnet, dass der obere Abschnitt (6) der Gegenspannscheibe (1) formschlüssig von unten in die Bohrung (21) der Knochenplatte (20) einführbar ist.

15. Fixationsvorrichtung nach einem der Ansprüche 9 - 14, dadurch gekennzeichnet, dass der obere Abschnitt (6) der Gegenspannscheibe (1) derart geformt ist, dass er drehfest in die Bohrung (21) der Knochenplatte (20) einführbar ist.

## Claims

1. Lock washer (1) for rigid fixation of bone screws (15) to a bone plate (20) which exhibits multiple bore holes (21), whereby the cylindrical lock washer (1) provides a central axis (2), a central bore hole (3) with an internal threading (4) that integrates with a bone screw (15), a lower section (5) with a bone adjoining surface (9) and an upper section (6), whereby in case of use of the lock washer (1) the upper section (6) is locked in the bore hole (21) of the bone plate (20), characterized in that the middle circumference of the lower section (5) is smaller than the middle circumference of the upper section (6).

2. Lock washer (1) according to claim 1, characterized in that the interior threading (4) is fully accommodated in the lower section (5).

3. Lock washer (1) according to one of the claims 1 or 2, characterized in that it tapers continuously or not continuously from the upper section (6) towards the lower section (5).

4. Lock washer (1) according to one of the claims 1 to 3, characterized in that the upper section (6) provides at least at the surface (7) which is adjacent to the bone plate (20) a roughness of structure to improve the torsional strength.

5. Lock washer (1) according to one of the claims 1 to 4, characterized in that it is shaped symmetrically.

6. Lock washer (1) according to one of the claims 1 to 4, characterized in that it is shaped asymmetrically.

7. Lock washer (1) according to one of the claims 1 the 6, characterized in that the surface (9) adjoining the bone is smaller than the average cross section being perpendicular to the axis (2) of the upper section (6).

8. Lock washer (1) according to one of claims 1 to 7, characterized in that it is shaped ellipsoidally.

9. A fixation device with at least one lock washer (1) according to one of the claims 1 to 8, a bone plate (20) with an underside (23) and an upper side (24), which are penetrated by bore holes (21), as well as at least one bone screw (15) with a head (16), a shaft (17) and an exterior threading (18), whereby in case of fixing the fixation device the upper section (6) of lock washer (1) can be brought to a stopped position in bore hole (21).

10. Fixation device according to claim 9, characterized in that at least one of the bore holes (21) provides a conical section (26) that widens toward the underside (23) which is shaped in form-locking fashion with the geometry of the upper section (6) of lock washer (1) and in case of fixing the fixation device the upper section (6) of the lock washer (1) is brought to a stopped position.

11. Fixation device according to claims 9 or 10, characterized in that at least one of the bore holes (21) of bone plate (20) provides at least at the surface that is adjacent to lock washer (1) a roughening or texturing to increase the torsional stability.

12. Fixation device according to claim 11, characterized in that the roughening or texturing is shaped in form of a tapering conical section (26) of the bore hole (21).

13. Fixation device according to one of the claims 9 to 12, characterized in that at least one of the bore holes (21) of bone plate (20) is at its narrowest width (29) equipped with securing pieces for lock washer (1) that are configured as elastic nipples (25).

14. Fixation device according to one of the claims 9 to 13, characterized in that the upper section (6) of the lock washer (1) is form-lockingly insertable from below into the bore hole (21) of the bone plate (20).

15. Fixation device according to one of the claims 9 to 14, characterized in that the upper section (6) of the lock washer (1) is shaped such that it is torsional securedly insertable into the bore hole (21) of the bone plate (20).

## Revendications

1. Rondelle de serrage (1) pour la fixation rigide de vis osseuses (15) sur une plaque osseuse (20) présentant plusieurs alésages (21), où la rondelle de serrage (1) cylindrique présentant un axe central (2), un alésage central (3) doté d'un filet interne (4) pouvant recevoir une vis osseuse (15), une partie inférieure (5) dotée d'une surface de pose (9) sur l'os et une partie supérieure (6), la partie supérieure (6) étant amenée à venir s'arrêter dans l'alésage (21) de la plaque osseuse (20) lorsque l'on utilise la rondelle de serrage, caractérisée en ce que le périmètre moyen de la partie inférieure (5) est plus petit que le périmètre moyen de la partie supérieure (6).

2. Rondelle de serrage (1) selon la revendication 1, caractérisée en ce que le filet interne (4) est entièrement disposé dans la partie inférieure (5).

3. Rondelle de serrage (1) selon les revendications 1 ou 2, caractérisée en ce qu'elle se rétrécit de manière continue ou non continue de la partie supérieure (6) vers la partie inférieure (5).

4. Rondelle de serrage (1) selon l'une des revendications 1 à 3, caractérisée en ce qu'au moins sur la surface (7) venant se poser contre la plaque osseuse (20), la partie supérieure (6) présente une rugosité ou une structuration pour augmenter la résistance à la rotation.

5. Rondelle de serrage (1) selon l'une des revendications 1 à 4, caractérisée en ce qu'elle est de forme symétrique.

6. Rondelle de serrage (1) selon l'une des revendications 1 à 4, caractérisée en ce qu'elle est de forme asymétrique.

7. Rondelle de serrage (1) selon l'une des revendications 1 à 6, caractérisée en ce que la surface d'appui (9) sur l'os est plus petite que la surface de la section transversale, perpendiculaire à l'axe (2), de la partie supérieure (6).

8. Rondelle de serrage (1) selon l'une des revendications 1 à 7, caractérisée en ce qu'elle présente la forme d'un ellipsoïde.

9. Dispositif de fixation comportant au moins une rondelle de serrage (1) selon l'une des revendications 1 à 8, une plaque osseuse (20) présentant une face inférieure (23) et une face supérieure (24), traversée par des alésages (21), ainsi qu'au moins une vis osseuse (15) dotée d'une tête (16), d'une tige (17) et d'un filet externe (18), tandis que lors de la fixation du dispositif de fixation, la partie supérieure (6) de la rondelle de serrage (1) vient s'arrêter dans l'alésage (21).

10. Dispositif de fixation selon la revendication 9, caractérisé en ce qu'au moins l'un des alésages (21) présente une partie conique (26) s'évasant en en direction de la face inférieure (23), dont la forme correspond géométriquement à la géométrie de la partie supérieure (6) de la rondelle de serrage (1) et qui, lors de la fixation du dispositif de fixation, vient se placer contre la partie supérieure (6) de la rondelle de serrage (1).

11. Dispositif de fixation selon les revendications 9 ou 10, caractérisé en ce qu'au moins sur sa surface en contact avec la rondelle de serrage (1), au moins l'un des alésages (21) de la plaque osseuse (20) est rendu rugueux ou reçoit une structuration pour augmenter la résistance à la rotation.

12. Dispositif de fixation selon la revendication 11, caractérisé en ce que la rugosité ou la structuration sont réalisées sous la forme d'une partie conique évasée (26) de l'alésage (21).

13. Dispositif de fixation selon l'une des revendications 9 à 12, caractérisé en ce qu'au moins l'un des alésages (21) de la plaque osseuse (20) est doté sur son périmètre le plus étroit (29) d'un élément de fixation de la rondelle de serrage (1), configuré comme mamelon élastique (25).

14. Dispositif de fixation selon l'une des revendications 9 à 13, caractérisé en ce que la partie supérieure (6) de la rondelle de serrage (1) peut être insérée en correspondance géométrique par le bas dans l'alésage (21) de la plaque osseuse (20).

15. Dispositif de fixation selon l'une des revendications 9 à 14, caractérisé en ce que la partie supérieure (6) de la rondelle de serrage (1) présente une forme telle qu'elle puisse être insérée dans l'alésage (21) de la plaque osseuse (20) de manière à ne pas pouvoir y tourner.
